# EUROPÄISCHE PATENTSCHRIFT

(11) **EP 0 765 861 B1**
(45) Veröffentlichungstag und Bekanntmachung des Hinweises auf die Patenterteilung: **18.08.1999**
(21) Anmeldenummer: 96115456.4
(22) Anmeldetag: 26.09.1996
(51) Int. Cl.: C07C 67/327, C07C 69/54

(54) **Verfahren zur Herstellung von Alkylestern der (Meth)acrylsäure**
Process for the preparation of alkyl esters of (meth)acrylic acid
Procédé de préparation d'esters alkyliques de l'acide (méth)acrylique

(30) Priorität: 28.09.1995 DE 19536183
(43) Veröffentlichungstag der Anmeldung: 02.04.1997
(73) Patentinhaber: BASF AKTIENGESELLSCHAFT, 67056 Ludwigshafen (DE)
(72) Erfinder: Baur, Karl Gerhard, Dr., 67063 Ludwigshafen (DE); Annen, Ulrich, Dr., 67454 Hassloch (DE); Exner, Herbert, Dr., 67165 Waldsee (DE); Fried, Michael, Dr., 69121 Heidelberg (DE); Rauh, Ulrich, 67117 Limburgerhof (DE)
(74) Vertreter: Isenbruck, Günter, Dr.

(56) Entgegenhaltungen:
- DE-A- 2 339 519
- DE-A- 19 547 485

## Beschreibung

Die Erfindung betrifft ein Verfahren zur Herstellung von Alkylestern der (Meth)acrylsäure aus Alkylestern von β-Acryloxy- oder β-Alkoxypropionsäure oder -isobuttersäure.

Im Verlauf der sauer katalysierten Herstellung von C₁- bis C₄-Alkylestern der (Meth)acrylsäure werden Verbindungen der nachstehenden allgemeinen Formel I, gegebenenfalls auch solche der allgemeinen Formel II als Nebenprodukt gebildet.

Die Erfindung betrifft insbesondere ein Verfahren zur Herstellung von C₁-bis C₄-Alkylestern der (Meth)acrylsäure ((Meth)acrylsäure steht für Acryl- oder Methacrylsäure) durch Eliminierung, d.h. Abspaltung, des Alkohols R²-OH aus Estern der allgemeinen Formel I 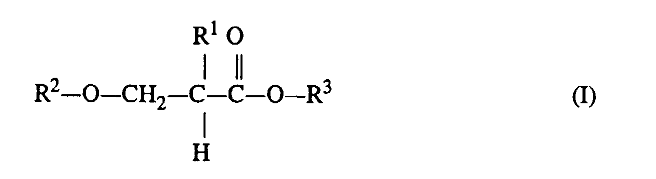
- in der R': Wasserstoff oder -CH₃ und
- R², R³: C₁- bis C₄-Alkyl bedeuten, in flüssiger Phase und in Gegenwart von Säure gemäß nachfolgendem Reaktionsschema: 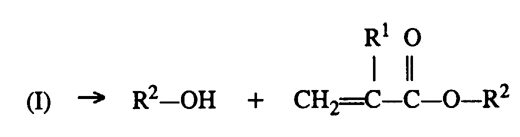 und gegebenenfalls entsprechende Eliminierung von (Meth)acrylsäure aus Estern der allgemeinen Formel II 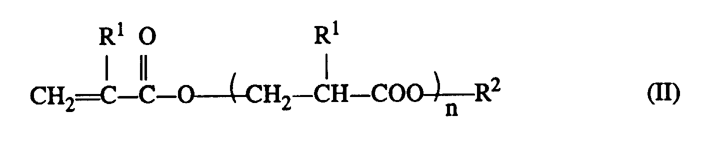
- in der R¹: Wasserstoff oder -CH₃,
- R²: C₁- bis C₄-Alkyl und
- n: eine ganze Zahl ≥ 0
bedeuten. Das heißt, noch nicht umgesetzter Alkohol und auch noch nicht umgesetzte (Meth)acrylsäure vermag sich an die Doppelbindung von bereits gebildetem (Meth)acrylsäurealkylester zu addieren, und wird erfindungsgemäß wieder eliminiert nach vorstehender Reaktion.

C₁- bis C₄-Alkylester der (Meth)acrylsäure sind allgemein bekannt. Sie bilden wichtige Ausgangsmonomere zur Herstellung von Polymerisaten, die beispielsweise in Form ihrer wäßrigen Dispersion in vielfacher Weise als Bindemittel Verwendung finden.

Die Herstellung von C₁- bis C₄-Alkylestern der (Meth)acrylsäure erfolgt üblicherweise durch Veresterung von (Meth)acrylsäure mit C₁- bis C₄-Alkanolen in flüssiger Phase und in Gegenwart von Säure als Katalysator (vgl. z.B. DE-A 23 39 519). Nachteilig an dieser Herstellweise ist, daß sich unter den vorgenannten Veresterungsbedingungen als Nebenreaktion noch nicht umgesetzter Ausgangsalkohol unter Ausbildung einer Verbindung der allgemeinen Formel I an die Doppelbindung von bereits gebildetem (Meth)acrylsäurealkylester addiert (Michael-Addition), wodurch die Bildung an gewünschtem Wertprodukt gemindert wird.

Aus der EP-A 379 691 und der EP-A 429 800 ist bekannt, (Meth)acrylsäurealkylester durch Alkoholabspaltung aus Alkylestern von β-Alkoxyalkancarbonsäuren in der Gasphase an kristallinen Zeolithen zu erzeugen. Nachteilig an dieser Verfahrensweise ist, daß sie die Durchführung in der Gasphase erfordert. Letzteres bedingt die beispielsweise die Anwendung vergleichsweise komplexer Rohr- bzw. Rohrbündelreaktoren. In gleicher Weise betreffen die DE-AS 11 26 378, die DE-AS 11 24 481, die DE-AS 11 24 482 und die DE-AS 11 24 483 die Spaltung von β-Alkoxymonocarbonsäureestern in Anwendung unterschiedlichster saurer Katalysatoren in der Gasphase.

Die DE-AS 23 39 519, die JP-A 05/25086, die JP-B 72/38419 und die CN-A 1 058 390 betreffen die Rückspaltung von β-Alkoxypropionsäurealkylestern in der Flüssigphase in Gegenwart von Säuren wie p-Toluolsulfonsäure, Schwefelsäure oder Phosphorsäure als Katalysator und in Anwendung von Drücken ≥ 1 atm. Nachteilig an dieser Verfahrensweise ist, daß ein erhöhter Anteil des abgespaltenen Alkohols zum entsprechenden Di-Alkylether weiterreagiert, wodurch einerseits auch bei Rückführung des Spaltproduktgemisches in die Veresterung derselben zu veresternder Alkohol entzogen und andererseits die Herstellung des (Meth)acrylsäurealkylesters mit Di-Alkylether belastet wird. So wird im Rahmen einer rektifikativen Abtrennung des Di-Alkylethers vom Veresterungsproduktgemisch in der Regel eine nicht unerhebliche Menge an Alkylacrylat, d.h. dem gewünschten Ester, mitgeschleppt. Demgegenüber weist das erfindungsgemäße Verfahren eine reduzierte Di-Alkylether-Bildung auf.

Das US-A 4 814 492 offenbart die Rückspaltung von C₁- bis C₅-Alkylestern der Methoxypropionsäure in die entsprechenden Alkylacrylate an mit Basen behandelten Zeolithen im Festbett und in Anwendung von Überdruck zur Aufrechterhaltung des flüssigen Aggregatzustandes. Gemäß der beispielhaften Ausführungsform des US-A 4 814 492 wird der abgespaltene Alkohol im Katalysatorfestbett absorbiert.

Nachteilig an dieser Verfahrensweise ist die nicht einfache Handhabung des Katalysatorfestbettes, das beispielsweise aufgrund von als Nebenreaktion einhergehender Polymerisatbildung von Zeit zu Zeit verstopft und ausgewechselt werden muß.

Spaltverfahren, die entsprechende Nachteile wie die vorgenannten Spaltverfahren aufweisen, offenbaren die CN-A 1 063 678, GB 923 595, JP-A 82/6229, JP-AS 72/15936 und die JP-A 94/65149.

Aufgabe der Erfindung war es daher, ein Verfahren zu schaffen, das die Nachteile der bekannten Verfahren vermeidet und insbesondere die vorgenannte Michael-Addition unter Rückbildung von erwünschtem (Meth)acrylsäurealkylester sowie Ausgangsalkohol umkehrt. Angestrebt wurde dabei insbesondere ein Spaltverfahren, dessen Produktgemisch als solches zur Erhöhung der Ausbeute an (Meth)acrylsäurealkylester unmittelbar in die Veresterungsstufe rückgeführt werden kann, ohne diese und die Aufarbeitung des dabei anfallenden Produktgemisches wesentlich zu beeinträchtigen.

Vor dem vorgenannten Stand der Technik als Hintergrund wurde erfindungsgemäß ein Verfahren zur Herstellung von C₁- bis C₄-Alkylestern der (Meth)acrylsäure aus Estern der allgemeinen Formel I 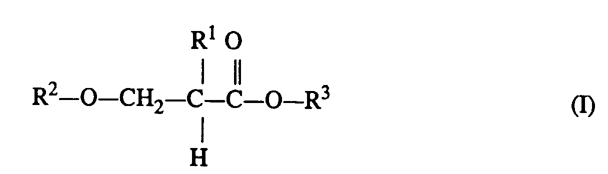 und gegebenenfalls estern der allgemeinen Formel (II) 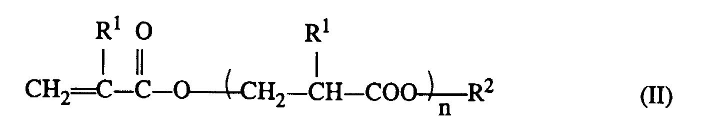 wobei
- R¹: Wasserstoff oder -CH₃ und
- R², R³: C₁- bis C₄-Alkyl und
- n: eine ganze Zahl ≥ 0 bedeuten
in flüssiger Phase und in Gegenwart von Säure bei reduziertem Druck (< 1 atm 1,013 bar) und unter kontinuierlicher Entnahme der Spaltprodukte gefunden.

Vorzugsweise sind R² und R³ identisch.

Von Bedeutung ist die Anwendung des erfindungsgemäßen Verfahrens vor allem bei Verbindungen I mit R², R³ = C₄-Alkyl, insbesondere n-Butyl. Mit Vorteil ist unter vorgenannten Voraussetzungen R¹ gleichzeitig H.

Ganz besonders vorteilhaft ist die Anwendung des erfindungsgemäßen Verfahrens im Fall von β-n-Butoxypropionsäure-n-butylester, als Verbindung (I) und β-Acryloxypropionsäure-n-butylester als Verbindung (II).

Als erfindungsgemäß geeignete katalytisch wirksame Säuren kommen sowohl Lewis-Säuren als auch Brönsted-Säuren in Betracht. Bei letzteren handelt es sich um Verbindungen, die an eine Brönsted-Base ein Proton abzugeben vermögen. Bei ersteren handelt es sich um Verbindungen, die eine Akzeptorstelle für Elektronenpaare aufweisen. Beispiele für Lewis-Säuren sind die Trihalogenide der dritten Hauptgruppe wie BF₃ oder AlCl₃. Vorzugsweise sind die katalytisch wirksamen Säuren in der anzuwendenden Menge unter den Reaktionsbedingungen in der Verbindung löslich. Es kommen aber auch fein verteilte Feststoffsäuren in Betracht. Beispiele dafür sind Aluminiumoxid oder Zirkoniumdioxid. Besonders geeignete Brönsted-Säuren sind die starken protischen Mineralsäuren. Als Beispiele seien genannt Schwefelsäure, Phosphorsäure, Methylsulfonsäure, Benzolsulfonsäure, Dodecylbenzolsulfonsäure und p-Toluolsulfonsäure. Vorzugsweise wird eine schwerflüchtige Mineralsäure angewendet. Es können auch saure Ionenaustauscherharze, z.B. sulfonsaure Harze, als Katalysatoren in im Reaktionsgemisch verteilter Form angewendet werden.

Die anzuwendende Menge der katalytisch wirksamen Säure hängt in dem Fachmann an sich bekannter Weise von der Art und Stärke der Säure ab. Im Fall von p-Toluolsulfonsäure werden, bezogen auf die Menge der zu spaltenden Verbindung I und gegebenenfalls II, mit Vorteil 1 bis 20 Gew.-%, vorzugsweise 5 bis 15 Gew.-% und besonders bevorzugt 5 bis 10 Gew.-% angewendet. Andere starke Säuren sind in vergleichbaren Mengen anzuwenden. Vorzugsweise wird das erfindungsgemäße Verfahren in Abwesenheit eines inerten Lösungsmittels durchgeführt.

Mit Vorteil liegt der Arbeitsdruck des erfindungsgemäßen Verfahrens bei 100 bis 900, vorzugsweise bei 200 bis < 500 und besonders bevorzugt bei 200 bis 400 mbar. Die Arbeitstemperatur liegt in der Regel bei 120 bis 220, vorzugsweise bei 160 bis 220 und besonders bevorzugt bei 180 bis 200 °C. Mit Vorteil liegt sie unterhalb des dem gewählten Arbeitsdruck entsprechenden Siedepunktes Tₛ des Reaktionsgemisches. Mit Vorteil gilt für die Arbeitstemperatur T_{A} des erfindungsgemäßen Verfahrens:

Tₛ-10 °C < T_{A} < Tₛ.

In zweckmäßiger Weise wird das erfindungsgemäße Verfahren in einem einfachen Rührkessel ausgeführt. Da die Siedepunkte der Spaltprodukte wesentlich niedriger liegen als der Siedepunkt der zu spaltenden Ausgangsverbindung, sind die Spaltprodukte (der Alkohol und der (Meth)acrylsäureester, gegebenenfalls auch (Meth)acrylsäure) in der mit der Flüssigphase im Gleichgewicht befindlichen Gasphase, relativ zur ersteren betrachtet, angereichert. Durch kontinuierliches Abtrennen der Gasphase können die Spaltprodukte daher in einfacher Weise dem Reaktionssystem entnommen werden.

Die Trennschärfe läßt sich dabei in einfacher Weise dadurch erhöhen, daß man zwischen Rührkessel und Entnahmestelle eine Rektifikationsvorrichtung schaltet, deren Rücklaufverhältnis in dem Fachmann an sich bekannter Weise so eingestellt wird, daß die Entnahme im wesentlichen ausschließlich die Spaltprodukte umfaßt. Als solche Rektifikationsvorrichtung kommen beispielsweise die an sich bekannten Rektifikationskolonnen wie z.B. eine Füllkörper-, Packungs- oder Bodenkolonne in Betracht.

Vorzugsweise wird das erfindungsgemäße Verfahren kontinuierlich ausgeführt, d.h. dem Reaktionskessel wird in dem Ausmaß zu spaltende Verbindung der allgemeinen Formel I und gegebenenfalls II kontinuierlich zugeführt, wie Spaltprodukte kontinuierlich entnommen werden. Mit Vorteil wendet man einen mit einem Zwangsumlaufverdampfer (z.B. Fallfilmverdampfer oder Flashverdampfer) gekoppelten Verweilzeitbehälter an. Bemerkenswerterweise mindert die erfindungsgemäße Verfahrensweise nicht nur die aus dem abgespaltenen Alkohol in einer Nebenreaktion gebildete Menge an Di-Alkylether. Vielmehr mindert sie auch die aus dem abgespaltenen Alkohol durch Dehydratisierung in einer weiteren Nebenreaktion gebildete Menge an Alken.

Die so kontinuierlich entnommenen Spaltprodukte können unmittelbar in die Veresterung zurückgeführt werden, beispielsweise in das Veresterungsgemisch oder eine der nachstehend beschriebenen Aufarbeitungsstufen.

Das erfindungsgemäße Verfahren ist insbesondere vorteilhaft für die sauer katalysierte Veresterung von n-Butanol mit Acrylsäure. Die Durchführung derselben erfolgt vorzugsweise bei Normaldruck oder reduziertem Druck in Anwendung einer Temperatur von 80 bis 135°C und eines geringen Überschusses an n-Butanol. In der Regel liegt das Molverhältnis von n-Butanol zu Acrylsäure bei 1,1:1. Die Veresterung erfolgt in Gegenwart von 1 bis 5 Gew.-%, bezogen auf die Reaktanden, an z.B. Schwefelsäure als Katalysator und in Anwendung eines üblichen leicht siedenden azeotropen Schleppmittels zur Entfernung des gebildeten Reaktionswassers in einer Rührkesselkaskade. Häufig enthält das im Rahmen der Veresterung anfallende Produktgemisch bis zu 10 Gew.-% an Verbindungen I und Verbindungen II. Charakteristisch ist, daß auch die Verbindungen II, wie die Verbindungen I, einen, im Vergleich zu den übrigen Bestandteilen des Veresterungsgemisches, erhöhten Siedepunkt aufweisen. Üblicherweise arbeitet man daher das Produktgemisch der Veresterung so auf, daß man durch Extraktion mit Wasser die katalytisch wirksame Säure separiert, anschließend mittels alkalischer wäßriger Lösung nicht umgesetzte (Meth)acrylsäure extrahiert, dann das Schleppmittel rektifikativ abtrennt. Sodann werden leichter als das Alkyl(meth)acrylat siedende Nebenprodukte (z.B. Di-Alkylether) rektifikativ entfernt, daraufhin das eigentliche Wertprodukt, das Alkyl(meth)acrylat, rektifikativ abgetrennt und das hauptsächlich aus Verbindungen I und II bestehende Sumpfprodukt wird entnommen.

Bemerkenswerterweise kann nun das Sumpfprodukt als solches dem erfindungsgemäßen Spaltverfahren zugeführt werden, da dieses nicht nur eine Rückführung der Verbindungen I in (Meth)acrylsäurealkylester und Alkohol bewirkt, sondern auch die Verbindungen II in (Meth)acrylsäure, (Meth)acrylsäurealkylester und gegebenenfalls Alkohol zurückführt.

Dieser Vorteil des erfindungsgemäßen Verfahrens kommt insbesondere im Rahmen der Herstellung von n-Butylacrylat zur Geltung, wo in typischer Weise Sumpfflüssigkeiten anfallen, die ≥ 50 Gew.-% (in der Regel 50 bis 60 Gew.-%) Butoxypropionsäurebutylester und ≥ 20 Gew.-% (in der Regel 20 bis 30 Gew.-%) Acryloxypropionsäurebutylester enthalten, die in Anwendung des erfindungsgemäßen Verfahrens wieder in die Wertprodukte n-Butanol, Acrylsäure und n-Butylacrylat ohne nennenswerte Verluste via Nebenreaktionen überführt und als solche in die Veresterung rückgeführt werden können.

Die Anwendung des erfindungsgemäßen Verfahrens erfolgt ebenso wie die eigentliche Veresterungsreaktion im Beisein üblicher Mengen üblicher Polymerisationsinhibitoren wie Phenothiazin, Hydrochinonmonomethylether oder Hydrochinon.

Abschließend ist festzustellen, daß das erfindungsgemäße Verfahren dadurch verbessert werden kann, daß dem Reaktionsgemisch bis zu 30, in der Regel 0,5 bis 25 Gew.-% seines Gewichts Wasser zugesetzt wird. Dies bedingt insbesondere eine erhöhte Raum/Zeit-Ausbeute an Spaltprodukt.

Die Erfindung betrifft auch ein Verfahren zur Herstellung eines C₁- bis C₄-Alkylesters der (Meth)acrylsäure durch sauer katalysierte Veresterung eines C₁- bis C₄-Alkanols mit (Meth)acrylsäure, dadurch gekennzeichnet, daß vom Produktgemisch die als Nebenprodukte entstehenden Verbindungen der allgemeinen Formel I sowie der allgemeinen Formel II abgetrennt werden, ihr Gemisch dem erfindungsgemäßen Verfahren unterworfen wird und die dabei kontinuierlich zu entnehmenden Spaltprodukte in die Veresterung zurückgeführt werden.

Weitere Einzelheiten und Vorteile der Erfindung können dem im folgenden beschriebenen Beispiel entnommen werden.

### BEISPIEL

Ein 2 l Doppelmantelrührbehälter wurde zur Hälfte seines Volumens mit einer im Rahmen der Herstellung von n-Butylacrylat anfallenden Sumpfflüssigkeit gefüllt, die nachfolgende Zusammensetzung aufwies:

| | |
|---|---|
| 0,5 Gew.-% | n-Butanol, |
| 6,5 Gew.-% | n-Butylacrylat; |
| 56,2 Gew.-% | n-Butoxypropionsäure-n-butylester, |
| 21,6 Gew.-% | Acryloxypropionsäure-n-butylester und |

als Restmenge höher kondensierte Nebenprodukte der Esterbildung sowie geringe Mengen radikalischer Oligomerisate und Phenothiazin als Polymerisationsinhibitor.

Zur Inertisierung wurden 5 l/h Stickstoff in das 2 1 Gefäß geführt. Auf den 2 1 Doppelmantelrührbehälter war eine 50 x 350 mm Packungsrektifikationskolonne aufgesetzt, die eine BX-Packung der Fa. Sulzer enthielt. Die Spalttemperatur betrug 195 °C. Als Säurekatalysator wurden 9 Gew.-%, bezogen auf das Reaktionsgemisch, p-Toluolsulfonsäure zugesetzt. Ein Teil (ca. 60 Gew.-%) des am Kopf der Rektifikationskolonne anfallenden Kondensats wurde in die Rektifikationskolonne rückgeführt. Der andere Teil wurde in einem Austragsbehälter gesammelt. Aus Stabilisierungsgründen wurden dem in die Rektifikationskolonne rückgeführten Kondensat, bezogen auf sein Gewicht, 800 ppm Phenothiazin als Polymerisationsinhibitor zugesetzt. Der Flüssigkeitsstand im Rührbehälter wurde durch kontinuierliche Ergänzung mit Sumpfflüssigkeit konstant gehalten.

Nach einer Betriebsdauer von 120 h wurde der Inhalt des Austragsbehälters gaschromatographisch analysiert. Nachfolgende Tabelle weist die Analysenergebnisse für 5 verschiedene Arbeitsdrücke aus (in Gew.-% bezogen auf die Gesamtmenge).

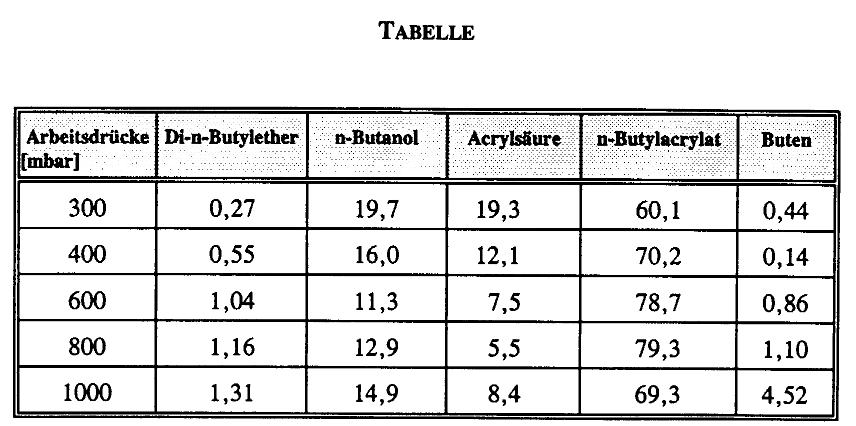

## Patentansprüche

1. Verfahren zur Herstellung von C₁- bis C₄-Alkylestern der (Meth)acrylsäure aus Estern der allgemeinen Formel I 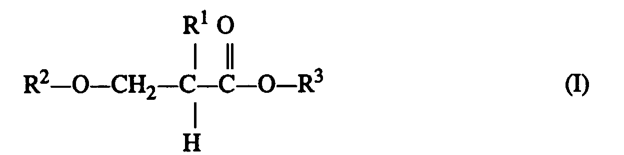 und gegebenenfalls Estern der allgemeinen Formel (II) 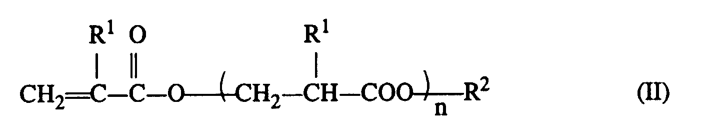 wobei
R¹ Wasserstoff oder -CH₃,
R², R³ C₁- bis C₄-Alkyl und
n eine ganze Zahl ≥ 0
bedeuten,
in flüssiger Phase und in Gegenwart von Säure bei reduziertem Druck und unter kontinuierlicher Entnahme der Spaltprodukte.

2. Verfahren nach Anspruch 1, dadurch gekennzeichnet, daß R² und R³ identisch sind.

3. Verfahren nach Anspruch 1 oder 2, dadurch gekennzeichnet, daß R² und R³ n-Butylreste sind.

4. Verfahren nach Anspruch 1 bis 3, dadurch gekennzeichnet, daß es sich bei der Verbindung (I) um β-n-Butoxypropionsäure-n-butylester und bei der Verbindung (II) um β-Acryloxypropionsäure-n-butylester handelt.

5. Verfahren nach Anspruch 1 bis 4, dadurch gekennzeichnet, daß als Säure p-Toluolsulfonsäure mitverwendet wird.

6. Verfahren nach Anspruch 1 bis 5, dadurch gekennzeichnet, daß der Arbeitsdruck 100 bis 900 mbar beträgt.

7. Verfahren nach Anspruch 1 bis 6, dadurch gekennzeichnet, daß die Arbeitstemperatur 120 bis 220 °C beträgt.

8. Verfahren nach Anspruch 1 bis 7, dadurch gekennzeichnet, daß ein zu spaltendes Reaktionsgemisch eingesetzt wird, das wenigstens 70 Gew.-%, bezogen auf seine Gesamtmasse, wenigstens einer Verbindung der allgemeinen Formel I und wenigstens einer Verbindung der allgemeinen Formel II enthält.

9. Verfahren nach einem der Ansprüche 1 bis 8, dadurch gekennzeichnet, daß das zu spaltende Reaktionsgemisch bis zu 30% seines Gewichts an Wasser enthält.

10. Verfahren zur Herstellung eines C₁- bis C₄-Alkylesters der (Meth)acrylsäure durch sauer katalysierte Veresterung eines C₁- bis C₄-Alkanols mit (Meth)acrylsäure, dadurch gekennzeichnet, daß vom Produktgemisch die als Nebenprodukte entstehenden Verbindungen der allgemeinen Formel I gemäß Anspruch 1 sowie der allgemeinen Formel II gemäß Anspruch 1 abgetrennt werden, ihr Gemisch einem Verfahren gemäß Anspruch 1 bis 9 unterworfen wird und die dabei kontinuierlich zu entnehmenden Spaltprodukte in die Veresterung zurückgeführt werden.

## Claims

1. A process for the preparation of C₁-C₄-alkyl esters of (meth)acrylic acid from esters of the formula I 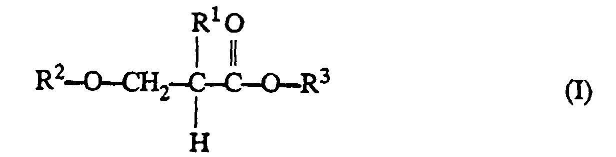 and, if appropriate, esters of the formula (II) 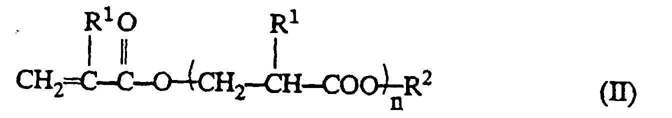 where
R¹ is hydrogen or -CH₃,
R² and R³ are each C₁-C₄-alkyl and
n is an integer ≥ 0,
in the liquid phase and in the presence of an acid at reduced pressure and with continuous removal of the cleavage products.

2. A process as claimed in claim 1, wherein R² and R³ are identical.

3. A process as claimed in claim 1 or 2, wherein R² and R³ are each n-butyl.

4. A process as claimed in any of claims 1 to 3, wherein the compound (I) is n-butyl β-n-butoxypropionate and the compound (II) is n-butyl β-acryloxypropionate.

5. A process as claimed in any of claims 1 to 4, wherein p-toluenesulfonic acid is concomitantly used as the acid.

6. A process as claimed in any of claims 1 to 5, wherein the operating pressure is from 100 to 900 mbar.

7. A process as claimed in any of claims 1 to 6, wherein the operating temperature is from 120 to 220°C.

8. A process as claimed in any of claims 1 to 7, wherein the reaction mixture to be cleaved comprises at least 70% by weight, based on its total weight, of at least one compound of the formula I and at least one compound of the formula II.

9. A process as claimed in any of claims 1 to 8, wherein the reaction mixture to be cleaved comprises up to 30% by weight of water.

10. A process for the preparation of a C₁-C₄-alkyl ester of (meth)acrylic acid by acid-catalyzed esterification of a C₁-C₄-alkanol with (meth)acrylic acid, which comprises separating off the compounds of the formula I as claimed in claim 1 and of the formula II as claimed in claim 1 which are formed as byproducts from the product mixture, subjecting their mixture to a process as claimed in any of claims 1 to 9 and recycling the cleavage products to be removed continuously to the esterification.

## Revendications

1. Procédé pour la production d'esters alkyliques en C₁ à C₄ de l'acide (méth)acrylique à partir d'esters de formule générale I 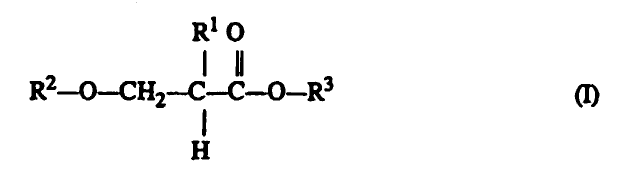 et éventuellement d'esters de formule générale II 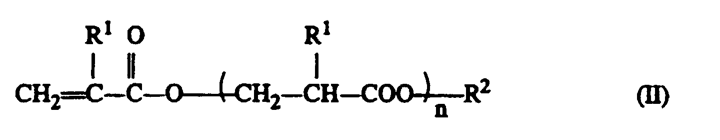 dans lesquelles
R¹ représente un atome d'hydrogène ou -CH₃,
R²,R³ représente un groupe alkyle en C₁ à C₄ et
n représente un nombre entier ≥ 0,
en phase liquide et en présence d'acide à une pression réduite et en retirant en continu les produits de dédoublement.

2. Procédé selon la revendication 1, caractérisé en ce que R² et R³ sont identiques.

3. Procédé selon la revendication 1 ou 2, caractérisé en ce que R² et R³ sont des résidus n-butyle.

4. Procédé selon les revendications 1 à 3, caractérisé en ce que, pour le composé (I), il s'agit de l'ester n-butylique de l'acide β-n-butoxypropionique et pour le composé (II) de l'ester n-butylique de l'acide β-acryloxypropionique.

5. Procédé selon les revendications 1 à 4, caractérisé en ce que l'on utilise conjointement comme acide, l'acide p-toluènesulfonique.

6. Procédé selon les revendications 1 à 5, caractérisé en ce que la pression de travail est de 100 à 900 mbars.

7. Procédé selon les revendications 1 à 6, caractérisé en ce que la température de travail est de 120 à 220°C.

8. Procédé selon les revendications 1 à 7, caractérisé en ce que l'on met en oeuvre un mélange réactionnel à dédoubler, qui contient au moins 70% en poids, par rapport à sa masse totale, d'au moins un composé de formule générale I et d'au moins un composé de formule II.

9. Procédé selon l'une quelconque des revendications 1 à 8, caractérisé en ce que le mélange réactionnel à dédoubler contient de l'eau à raison de jusqu'à 30% de son poids.

10. Procédé pour produire un ester alkylique en C₁ à C₄ de l'acide (méth)acrylique au moyen d'une estérification catalysée par un acide, d'un alcanol en C₁ à C₄ avec de l'acide (méth)acrylique, caractérisé en ce que les composés créés en tant que sous-produits de formule générale I selon la revendication 1 ainsi que de formule générale II selon la revendication 1 sont séparés d'avec le mélange de produits, leur mélange est soumis à un procédé selon les revendications 1 à 9 et les produits de dédoublement qui sont retirés en continu, sont recyclés dans l'estérification.
